# EUROPEAN PATENT APPLICATION

(11) **EP 2 484 337 A2**
(43) Date of publication of application: **08.08.2012**
(21) Application number: 12153794.8
(22) Date of filing: 03.02.2012
(51) Int. Cl.: A61K 8/25, A61Q 15/00

(54) **Treatment of sweating**

(30) Priority: 04.02.2011 US 201161439513 P
(71) Applicant: Janssen Pharmaceuticals, Inc., Titusville, NJ 08560 (US)
(72) Inventor: Frentzko, Michael J., Sussex, NJ New Jersey 07461 (US); Kendall, Clare S. L., Mendham, NJ New Jersey 07945 (US); Newburger, Joan, West Trenton, NJ New Jersey 08628 (US); Revankar, Ratna, Princeton, NJ New Jersey 08540 (US); Shana'a, May, Martinsville, NJ New Jersey 08836 (US)
(74) Representative: Warner, James Alexander

(57) **Abstract**

A method of treating non-pathological or pathological sweating is provided, which comprises topically applying to skin in need of treatment for sweating an occlusive composition, for example comprising silicone.

## Description

### FIELD OF THE INVENTION

The present invention relates to compositions, devices and methods for the treatment of sweating, including non-pathological and pathological or excessive sweating, such as hyperhidrosis, via the topical application of an occlusive composition, for example containing a silicone. Preferably, the composition is substantially free of active ingredients commonly used to alleviate or lesson sweating.

### BACKGROUND OF THE INVENTION

The physiological act of sweating serves as the body's natural coolant, protecting it from overheating. When subjected to temperature increases, stress, or exercise, the body excretes sweat, a fluid comprising mostly water along with minerals, lactate and urea, to cool the body by evaporation of the water.

In people with excessive sweating or hyperhidrosis, this function is overactive and they experience extreme sweating, more than is usual or necessary. This disorder can be uncomfortable, it can cause significant embarrassment and it can be emotionally debilitating. Excess sweating can occur on the palms of the hands, soles of the feet, underarms, face, head, or a combination of these sites or other sites on the body. Excessive sweating can lead to further dermatological disorders and social stigma. It is estimated that approximately two percent of the population suffers from hyperhidrosis.

Conventional treatments for hyperhidrosis include the use of antiperspirants, aluminum chloride, botulinum toxin injections, surgical procedures such as extrathoracic sympathectomy and electrical stimulation via tap water iontophoresis. Iontophoretic devices for the treatment of hyperhidrosis are described in example U.S. Patent Appln. Publication No. 2004/0167461 to Nitzan et al. and U.S. Patent No. 6,223,076 to Tapper. WO 2010/027792 discloses the treatment of hyperhidrosis using galvanic particulates. US 2009/0232746 discloses a base formulation system for antiperspirants comprising an amidomethicone, dimethicone gum, and silica.

Even normal, non-pathological sweating can be undesirable, for example on the hands or the torso. Over the counter antiperspirants are typically for use only on the underarms and can be irritating or otherwise less than optimal or effective for normal sweating. They can be ineffective in treating hyperhidrosis. Higher strength antiperspirants prescribed for the hyperhidrosis can also cause significant irritation. Surgical sypathectomy can cause unwanted compensatory sweating in other regions of the body. Botulinum toxin injections are very painful when used for treating palmar hyperhidrosis. Conventional tap water iontophoretic devices like the above are less than optimal. They are inconvenient to use, and immobilize the patient during treatment. They are also require the use of relatively high electric currents, around 18 milliamps, and which may, depending on the design, be directed through major portions of the body remote from the treatment area, including passing through the heart. They are typically painful for the person undergoing treatment due to the high current. This is a particular problem in that treatment often requires several sessions per week over a period of weeks or months.

Applicants have now discovered that topical application of occlusive compositions, especially those containing one or more silicones, preferably silicones without nitrogen-containing functional groups, provide relief from non-pathological sweating and pathological sweating such as hyperhidrosis.

### SUMMARY OF THE INVENTION

In one aspect, the invention features a method of treating sweating, which comprises topically applying to skin in need of treatment for sweating a composition comprising silicone.

In another aspect, the invention provides a method of treating sweating, which comprises topically applying to skin in need of treatment for sweating an occlusive composition.

### DETAILED DESCRIPTION OF THE INVENTION

It is believed that one skilled in the art can, based upon the description herein, utilize the present invention to its fullest extent. The following specific embodiments are merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

As used herein, a composition that is "substantially free" of an ingredient means the composition that has about 2% or less of that ingredient by weight based on the total weight of the composition. Preferably, a composition that is substantially free of an ingredient has about 1% or less, more preferably about 0.5% or less, more preferably about 0.1% or less, more preferably about 0.05 or less, more preferably about 0.01% or less by weight based on the total weight of composition of the ingredient. In certain more preferred embodiments, a composition that is substantially free of an ingredient is free of the ingredient, i.e. has none of that ingredient in the composition.

As used herein, "cosmetically-acceptable" means that the ingredients which the term describes are suitable for use in contact with tissues (e.g., the skin or hair) without undue toxicity, incompatibility, instability, irritation, allergic response, and the like.

As used herein, "safe and effective amount" means an amount of the ingredient or of the composition sufficient to provide the desired benefit at a desired level, but low enough to avoid serious side effects. The safe and effective amount of the ingredient or composition will vary with the area being treated, the age and skin type of the end user, the duration and nature of the treatment, the specific ingredient or composition employed, the particular cosmetically-acceptable topical carrier utilized, and like factors.

As used herein, the term "treatment" means the alleviation or elimination of symptoms, and/or cure, and/or prevention or inhibition of a disease or condition, specifically sweating.

As used herein, "sweating" refers to the excretion of perspiration from the pores of the skin. Sweating includes, but not limited to, 1) non-pathological sweating, such as thermally-induced, exercise-induced, or stress-induced sweating; and 2) pathological or excessive sweating such as hyperhidrosis, including primary and secondary hyperhidrosis, as well as focal and generalized hyperhidrosis. The invention is useful in any area of the body where sweating occurs such as the hands, feet, face, head, torso, or underarms (axilla).

According to the invention, sweating may be treated by topical application of an occlusive composition to skin in need of treatment for sweating.

### Composition

In one embodiment, the composition comprises at least one silicone. Preferably, the silicone is free of nitrogen-containing functional groups.

An example of a suitable composition is a blend comprising cyclopentasiloxane, dimethicone, dimethicone crosspolymer, trisiloxane, silica and dimethicone/vinyl dimethicone crosspolymer. Such a blend is commercially available as DOW CORNING® CF-0055 Custom Blend.

The composition is preferably applied in a safe and effective amount for the treatment of sweating. For example, the amount of a composition comprising a silicone applied to the palm of the hand is preferably about one-half the size of a pea. This amount may be applied several times during the day, for example once in the morning, then throughout the day after each washing the hands or other removal of the composition, and then again at bedtime. Alternatively, the composition may be applied twice a day, in the morning and at bedtime, or applied once a day at bedtime.

In one embodiment, the composition is substantially free, preferably free, of active agents for sweat reduction such as antiperspirants or anticholinergic drugs. Antiperspirants include, but are not limited to, aluminum salts such as aluminum chloride and aluminum chlorohydrate, and aluminum zirconium compounds such as aluminum zirconium tetrachlorohydrex gly. Anticholinergic drugs include but are not limited to oxybutynin, glycopyrrolate, propantheline bromide, benzatropine, and botulinum neurotoxins (BOTOX®), including BOTOX® analogs, BOTOX® active fragments, and natural BOTOX® active metabolites.

The composition may be applied to the treatment area in a variety of forms including but not limited to gels, creams, or sticks. In one embodiment, the composition comprises a cosmetically-acceptable topical carrier.

As will be recognized by those of skill in the art, cosmetically-acceptable topical carriers comprise carriers that are suitable for use in contact with the body, in particular the skin for treatment of sweating, without undue toxicity, incompatibility, instability, irritation, allergic response, and the like. A safe and effective amount of carrier is from about 50% to about 99.999%, preferably from about 80% to about 99.9%, more preferably from about 99.9% to about 95%, most preferably from about 99.8% to about 98% of the composition. The carrier can take a wide variety of forms. For example, emulsion carriers, including, but not limited to, oil-in-water, water-in-oil, water-in-oil-in-water, and oil-in-water-in-silicone emulsions, are useful herein. These emulsions can cover a broad range of viscosities, e.g., from about 100 cps to about 200,000 cps. Examples of suitable cosmetically-acceptable topical carriers include cosmetically-acceptable solvents and materials for cosmetic solutions, suspensions, lotions, creams, serums, essences, gels, toners, sticks, sprays, ointments, liquid washes and soap bars, shampoos, hair conditioners, pastes, foams, mousses, powders, shaving creams, wipes, patches, strips, powered patches, microneedle patches, bandages, hydrogels, film-forming products, facial and skin masks, make-up, liquid drops, and the like. These product types may contain several types of cosmetically-acceptable topical carriers including, but not limited to solutions, suspensions, emulsions such as microemulsions and nanoemulsions, gels, solids, liposomes, other encapsulation technologies and the like. The following are non-limitative examples of such carriers. Other carriers can be formulated by those of ordinary skill in the art.

In one embodiment, the carrier contains water. In a further embodiment, the carrier may also contain one or more aqueous or organic solvents. Examples of organic solvents include, but are not limited to: dimethyl isosorbide; isopropylmyristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (e.g., from about 2 carbon atoms to about 4 carbon atoms), such as isopropanol and ethanol. Examples of polyols include, but are not limited to, those having from about 2 carbon atoms to about 15 carbon atoms (e.g., from about 2 carbon atoms to about 10 carbon atoms) such as propylene glycol. The organic solvents may be present in the carrier in an amount, based upon the total weight of the carrier, of from about 1 percent to about 99.99 percent (e.g., from about 20 percent to about 50 percent). Water may be present in the carrier (prior to use) in an amount, based upon the total weight of the carrier, of from about 5 percent to about 95 percent (e.g., from about 50 percent to about 90 percent). Solutions may contain any suitable amounts of solvent, including from about 40 to about 99.99%. Certain preferred solutions contain from about 50 to about 99.9%, from about 60 to about 99%, from about 70 to about 99%, from about 80 to about 99%, or from about 90 to 99%.

A lotion can be made from such a solution. Lotions typically contain at least one emollient in addition to a solvent. Lotions may comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s).

The compositions useful in the present invention can also be formulated as emulsions. If the carrier is an emulsion, from about 1% to about 10% (e.g., from about 2% to about 5%) of the carrier contains an emulsifier(s). Emulsifiers may be nonionic, anionic or cationic.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or wipe). The composition of the present invention can also be combined with a solid, semi-solid or dissolvable substrate (e.g., a wipe, mask, pad, glove or strip).

The composition is applied topically by simple rubbing with the fingers, spraying, or with other applicators known in the art of topical compositions, such as, but not limited to, a pad or wipe.

In one embodiment, the composition contacts substantially all, that is, at least 80 percent, preferably 90 percent, of the surface area of the skin in need of treatment. More preferably, the composition contacts all, that is, 100 percent, of the surface area of the skin in need of treatment.

### Garment

In one embodiment, the composition is used in conjunction with a garment. For example, users may apply the composition to the treatment area followed by covering with a garment to preserve the occlusive environment, intensify treatment, and/or prevent the composition from unintentionally rubbing off. For example, for the treatment of palmar sweating, a patient may apply the composition to the palm followed by donning a nitrile glove. Alternatively, the composition may be contained in the garment by incorporating the composition onto the surface of the garment.

The garment may be fabricated into various shapes and sizes to fit the contours of various anatomical surfaces of the body. For example, it may be a glove, a sock, a hat, tights, a wrap, a cuff, a band such as an armband or leg band or headband, a shoe, a shoe insert, a belt, a vest, or a shirt. In particular, the garment may be a glove or a sock for treatment of the palm of the hand or sole of the foot, respectively. For treatment of the axilla, the garment may consist of an underarm strap.

Preferably, the garment fits the treatment area snugly to provide good contact between the occlusive composition and the skin of the treatment area.

The garment may be made of a variety of materials. Examples of materials for use in or as the garment include, but are not limited to: cotton-based gauze; non-woven pads made of rayon or a mixture of rayon, polyester and/or other polymer fibers; felts, woven fabrics, conductive nonwoven and woven materials, open-cell foam and sponge-like materials contained of polyurethane, polyester and/or other polymers; and cross-linked and noncross-linked gelling materials, such as polyacrylamide, polyvinyl alcohol, gelatin, hydroxymethy1cellulose, hydroxyethylcellulose, hydroxypropylcellulose, methylcellulose, and carboxymethy1cellulose.

Examples of further materials for use in or as the garment include, but are not limited to, cross-linked and non-cross-linked polymers; swellable polymers such as water-swollen cellulose derivatives (e.g., methylcellulose (MC), hydroxyethyl methylcellulose (HEMA), hydroxypropyl methylcellulose (HPMC), ethylhydroxyethyl cellulose (EHEC), hydroxyethylcellulose (HEC), hydroxypropylcellulose (HPC), and carboxymethlcellulose (CMC) and their salts); polyvinyl alcohol (PVA); polyvinylpyrrolidone (PVP); polyethylene oxide (PEO); polymers prepared by monomers such as hydroxyethyl methacrylate (HEMA), hydroxyethoxyethyl methacrylate (HEEMA), hydroxydiethoxyethyl methacrylate (HDEEMA), methyoxyethyl methacrylate (MEMA), methoxyethoxyethyl methacrylate (MEEMA), methyldiethoxyethyl methacrylate (MDEEMA), ethylene glycol dimethacrylate (EGDMA), n-vinyl- 2pyrrolidone (NVP), methacrylic acid (MA), and vinyl acetate (VAC); polycrylamide, polyacrylate polymers, cross-linked and non-cross-linked polyacrylic acids of various molecular weight and their salts (sodium, potassium, magnesium, calcium, aluminum, etc.); gelatin; gums and polysaccharides such as gum arabic, gum karaya, gum tragacanth, guar gum, gum benzoin, and alginic acid and their salts; polyethylene glycol (PEG); polypropylene glycol (PPG); and clays or other swellable minerals such as bentonite and montmorillonite.

In one embodiment, the material comprises a non-woven material. By "non-woven" is meant that the material, or a layer of the material, is comprised of fibers that are not woven into a fabric but rather are formed into a sheet, mat, or pad layer. The fibers can either be random (i.e., randomly aligned) or they can be carded (i.e., combed to be oriented in primarily one direction. Furthermore, the non-woven material can be composed of a combination of layers of random and carded fibers).

Non-woven materials may be comprised of a variety of natural and/or synthetic materials. By "natural" is meant that the materials are derived from plants, animals, insects, or byproducts of plants, animals, and insects. By "synthetic" is meant that the materials are obtained primarily from various man-made materials or from natural materials, which have been further altered.

Non-limiting examples of natural materials useful in the present invention are silk fibers, keratin fibers (such as wool fibers, camel hair fibers) and cellulosic fibers (such as wood pulp fibers, cotton fibers, hemp fibers, jute fibers, and flax fibers).

Examples of synthetic materials include, but are not limited to, those selected from the group containing acetate fibers, acrylic fibers, cellulose ester fibers, cotton fibers, modacrylic fibers, polyamide fibers, polyester fibers, polyolefin fibers, polyvinyl alcohol fibers, rayon fibers, polyurethane foam, and mixtures thereof.

Materials made from one or more of the natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), and Fort James Corporation (Deerfield, IL USA).

Methods of making non-woven materials are also well known in the art. Such methods include, but are not limited to, air-laying, water-laying, melt-blowing, spin-bonding, or carding processes. The resulting material, regardless of its method of production or composition, is then subjected to at least one of several types of bonding operations to anchor the individual fibers together to form a self-sustaining web. The non-woven substrate can be prepared by a variety of processes including hydro-entanglement, thermally bonding, and combinations of these processes. Moreover, the materials can have a single layer or multiple layers. In addition, a multilayered material can include film layer(s) (e.g., aperture or non-aperture film layers) and other non-fibrous materials.

Strength, thickness, or firmness of the non-woven material may be a desirable attribute. This can be achieved, for example, by the addition of binding materials, such as wet strength resins, or the material may be made of polymer binder coatings, stable fibres, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the substrate may range from about 5% to about 20%, by weight, of the substrate.

Non-woven materials of increased strength can also be obtained by using the so-called spunlace or hydro-entanglement technique. In this technique, the individual fibers are twisted together so that an acceptable strength or firmness is obtained without the need to use binding materials. The advantage of the latter technique is the excellent softness of the non-woven material.

Additives may also be added in order to increase the softness of the substrates. Examples of such additives include, but are not limited to, polyols such as glycerol, propylene glycol and polyethylene glycol, phthalate derivatives, citric esters, surfactants such as polyoxyethylene (20) sorbitanesters, and acetylated monoglycerides.

Barriers may be incorporated into the garments to prevent leakage of the composition from the device, which may result in loss of efficacy. They may consist of impermeable films, such as polymer or latex films, or barrier treatments on the garment's surface.

Sensory attributes may also be incorporated to the insoluble non-woven substrates. Examples of such sensory attributes include, but are not limited to color, texture, pattern, and embossing.

### Other Ingredients

In one embodiment, the composition contains one or more other ingredients typically used in topical compositions, many of which are familiar to those skilled in the art. For example, the composition may contain a plant extract, anti-inflammatory agent, antimicrobial agent, fragrance, or other active agent, preferably an active agent not used to treat sweating.

Examples of plant extracts include, but are not limited to, feverfew, soy, glycine soja, oatmeal, what, aloe vera, cranberry, witch-hazel, alnus, arnica, artemisia capillaris, asiasarum root, birch, calendula, chamomile, cnidium, comfrey, fennel, galla rhois, hawthorn, houttuynia, hypericum, jujube, kiwi, licorice, magnolia, olive, peppermint, philodendron, salvia, sasa albo- marginata, natural isoflavonoids, soy isoflavones, and natural essential oils.

In one embodiment, the composition contains ingredients to alleviate or prevent skin irritation and inflammation. These ingredients may be natural extracts, such as but not limited to feverfew, aloe, or chamomile. In one embodiment these ingredients may include topical steroids including corticosteroids, such as hydrocortisone. In one embodiment, the composition may include nonsteroidal anti-inflammatory agents.

In one embodiment, the composition contains a buffering agent such as citrate buffer, phosphate buffer, lactate buffer, gluconate buffer, or gelling agents, thickeners, or polymers.

In one embodiment, the composition contains a fragrance, for example one effective for reducing stress, calming, and/or affecting sleep such as lavender and chamomile.

The following non-limiting example further illustrates the invention.

### Example

An eight week clinical study for the treatment of hyperhidrosis on the palms was conducted using topical application of a formulation containing an active constituent and inert, silicone-based excipients or a formulation containing only the inert, silicone-based excipients (vehicle formulation). The vehicle formulation was DOW CORNING® CF-0055 Custom Blend.

In total, nineteen (19) subjects were asked to apply the test product containing only the inert, silicone-based excipients (vehicle formulation) to the palms, rubbing it into the palms in a manner similar to the application of a moisturizer. They were instructed not to apply the product to the back of the hands. A small amount of the test product, approximately half the size of a pea, was used at each application. Subjects applied the product to their palms in the morning, and re-applied the product throughout the day following routine hand-washing, or if they felt that they had otherwise inadvertently wiped the product off of their hands with excessive wiping or rubbing. (They were instructed to wash the product off before eating or preparing food, and reapply the product afterwards.) They also applied the product again at night before sleep.

Self-assessment at eight weeks was measured versus baseline using the Hyperhidrosis Disease Severity Scale:

| **Score** | **Hyperhidrosis Disease Severity Scale** |
|---|---|
| 1 | My sweating is never noticeable and never interferes with my daily activities |
| 2 | My sweating is tolerable but sometimes interferes with my daily activities |
| 3 | My sweating is barely tolerable and frequently interferes with my daily activities |
| 4 | My sweating is intolerable and always interferes with my daily activities |

As a study entry criterion, the subjects needed to assess themselves at baseline as a Grade 4 or Grade 3 on the Hyperhidrosis Severity Scale.

The rate of improvement in the 19 silicone vehicle formulation-treated subjects versus baseline was unexpectedly high. Specifically, six of the nine subjects (67%) using the silicone vehicle formulation who started out at a Grade 4 on the Hyperhidrosis Disease Severity Scale saw a 2 grade improvement. Eighty-four percent of the silicone vehicle formulation-treated subjects had an improvement from baseline of one or more grades, and 42% of the silicone vehicle formulation-treated subjects had an improvement from baseline of two or more grades. A spontaneous sizeable reduction of sweating in hyperhidrosis patients is unexpected.

### FURTHER EMBODIMENTS OF THE INVENTION

1. A method of treating sweating, which comprises topically applying to skin in need of treatment for sweating a composition comprising silicone.
2. The method of embodiment 1, wherein said composition is substantially free of active agents for sweat reduction.
3. The method of embodiment 1 or 2, wherein the silicone is free of nitrogen-containing functional groups.
4. The method of any one of embodiments 1 to 3, wherein said composition comprises a cosmetically-acceptable topical carrier.
5. The method of any one of embodiments 1 to 4, wherein said sweating is non-pathological.
6. The method of any one of embodiments 1 to 4, wherein said sweating is pathological.
7. A method of treating sweating, which comprises topically applying to skin in need of treatment for sweating an occlusive composition.
8. The method of embodiment 7, wherein said composition is substantially free of active agents for sweat reduction.
9. The method of embodiment 7 or 8, wherein said composition comprises a cosmetically-acceptable topical carrier.
10. The method of any one of embodiments 7 to 9, wherein said sweating is non-pathological.
11. The method of any one of embodiments 7 to 9, wherein said sweating is pathological.

## Claims

1. A method of treating sweating, which comprises topically applying to skin in need of treatment for sweating a composition comprising silicone.

2. The method of claim 1, wherein said composition is substantially free of active agents for sweat reduction.

3. The method of claim 1, wherein the silicone is free of nitrogen-containing functional groups.

4. The method of claim 1, wherein said composition comprises a cosmetically-acceptable topical carrier.

5. The method of claim 1, wherein said sweating is non-pathological.

6. The method of claim 1, wherein said sweating is pathological.

7. A method of treating sweating, which comprises topically applying to skin in need of treatment for sweating an occlusive composition.

8. The method of claim 7, wherein said composition is substantially free of active agents for sweat reduction.

9. The method of claim 7, wherein said composition comprises a cosmetically-acceptable topical carrier.

10. The method of claim 7, wherein said sweating is non-pathological.

11. The method of claim 7, wherein said sweating is pathological.

12. A composition comprising silicone for use in a method of treating sweating of any one of claims 1 to 6.

13. Silicone for use in a method of treating sweating of any one of claims 1 to 6.

14. An occlusive composition for use in a method of treating sweating of any one of claims 7 to 11.
